(19) 

Europäisches
Patentamt
European
Patent Office
Office européen
des brevets

(11) **EP 4 417 308 A1**

(12) **EUROPEAN PATENT APPLICATION**
published in accordance with Art. 153(4) EPC

(43) Date of publication:
**21.08.2024 Bulletin 2024/34**

(21) Application number: **23887155.2**

(22) Date of filing: **13.09.2023**

(51) International Patent Classification (IPC):
**B01J 35/00** $^{(2024.01)}$        **B01J 23/10** $^{(2006.01)}$
**B01J 23/80** $^{(2006.01)}$        **B01J 37/02** $^{(2006.01)}$
**B01J 37/03** $^{(2006.01)}$        **B01J 21/04** $^{(2006.01)}$
**B01J 21/08** $^{(2006.01)}$        **B01J 21/06** $^{(2006.01)}$
**B01J 37/08** $^{(2006.01)}$

(52) Cooperative Patent Classification (CPC):
**B01J 21/04; B01J 21/06; B01J 21/08; B01J 23/10;
B01J 23/80; B01J 35/00; B01J 37/02; B01J 37/03;
B01J 37/08; C07C 5/48; C07C 11/167**

(86) International application number:
**PCT/KR2023/013694**

(87) International publication number:
**WO 2024/111832 (30.05.2024 Gazette 2024/22)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **23.11.2022 KR 20220158634**

(71) Applicant: **LG Chem, Ltd.**
**Yeongdeungpo-gu**
**Seoul 07336 (KR)**

(72) Inventors:
• **KIM, Hanna**
  **Daejeon 34122 (KR)**
• **LEE, Dong Min**
  **Daejeon 34122 (KR)**
• **JEOUNG, Jaekwon**
  **Daejeon 34122 (KR)**
• **CHA, Kyong Yong**
  **Daejeon 34122 (KR)**
• **HAN, Sang Jin**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **CATALYST FOR OXIDATIVE DEHYDROGENATION, AND PREPARATION METHOD THEREFOR**

(57)    A catalyst for an oxidative dehydrogenation reaction according to an exemplary embodiment of the present application comprises: a carrier; a first coating layer provided on the carrier and comprising a metal oxide; and a second coating layer provided on the first coating layer and comprising a zinc ferrite-based catalyst, in which the metal oxide comprises one or more metals selected from among Ce, K, Mg, La and Y.

[Figure 1]

**EP 4 417 308 A1**

**Description**

[Technical Field]

This application claims priority to and the benefit of Korean Patent Application No. 10-2022-0158634 filed in the Korean Intellectual Property Office on November 23, 2022, the entire contents of which are incorporated herein by reference.

The present application relates to a catalyst for an oxidative dehydrogenation reaction and a method for producing the same.

[Background Art]

1,3-butadiene is an intermediate of petroleum chemical products, and demands for 1,3-butadiene and the value thereof are gradually increasing globally. The 1,3-butadiene has been manufactured using naphtha cracking, the direct dehydrogenation reaction of butene, the oxidative dehydrogenation reaction of butene, and the like.

However, since the naphtha cracking process consumes a lot of energy due to high reaction temperature and is not a single process for producing only 1,3-butadiene, there is a problem in that other fundamental fractions in addition to 1,3-butadiene are produced in excess. Further, the direct dehydrogenation reaction of n-butene is thermodynamically adverse and requires high temperature and low pressure conditions for manufacturing 1,3-butadiene with high yield as an endothermic reaction, and thus is not suitable as a commercialization process for producing 1,3-butadiene.

Meanwhile, the oxidative dehydrogenation reaction of butene is a reaction in which butene and oxygen react with each other in the presence of a metal oxide catalyst to manufacture 1,3-butadiene and water, and has a very thermodynamically favorable advantage because stable water is produced. Further, since the oxidative dehydrogenation reaction of butene is an exothermic reaction unlike the direct dehydrogenation reaction of butene, 1,3-butadiene with high yield may be obtained even at lower reaction temperature than the direct dehydrogenation reaction, and the oxidative dehydrogenation reaction of butene may become an effective single production process capable of satisfying the demands for 1,3-butadiene because an additional heat supply is not required.

The metal oxide catalyst is generally synthesized by a precipitation method, and since the one-time amount of metal oxide catalyst produced is small due to technical and spatial limitations, the catalyst is prepared by repeating the same process several times in order to satisfy a target amount. The catalysts thus prepared over several times may have different reactivity with the reactant depending on the preparation order, and such a difference in reactivity among the catalysts is directly related to the yield of the product (butadiene), so that studies to reduce the difference in reactivity among the catalysts have been continuously carried out.

[Detailed Description of the Invention]

[Technical Problem]

The present application has been made in an effort to provide a catalyst for an oxidative dehydrogenation reaction, which is capable of obtaining butadiene with a high yield by increasing the conversion rate of butene and increasing the selectivity of butadiene, and a method for producing the same.

[Technical Solution]

An exemplary embodiment of the present application provides a catalyst for an oxidative dehydrogenation reaction, comprising:

a carrier;
a first coating layer provided on the carrier and comprising a metal oxide; and
a second coating layer provided on the first coating layer and comprising a zinc ferrite-based catalyst,
in which the metal oxide comprises one or more metals selected from among Ce, K, Mg, La and Y.

Further, another exemplary embodiment of the present application provides a method for producing a catalyst for an oxidative dehydrogenation reaction, the method comprising:

forming a first coating layer by coating a carrier with a first solution comprising a metal oxide precursor, and then performing a first heat treatment process; and
forming a second coating layer by coating the first coating layer with a second solution comprising a zinc ferrite-based catalyst, and then performing a second heat treatment process,
in which the metal oxide precursor comprises one or more metals selected from among Ce, K, Mg, La and Y.

Further, still another exemplary embodiment of the present application provides a method for producing butadiene, the method comprising:

preparing the catalyst for an oxidative dehydrogenation reaction; and
producing butadiene using the catalyst for an oxidative dehydrogenation reaction in an oxidative dehydrogenation reaction of butene.

[Advantageous Effects]

A catalyst for an oxidative dehydrogenation reaction according to an exemplary embodiment of the present application can be provided with a first coating layer comprising a metal oxide and a second coating layer comprising a zinc ferrite-based catalyst on a carrier in this order to suppress a phenomenon in which coke is deposited on the catalyst and increase the activity of the catalyst.
Therefore, the catalyst for an oxidative dehydrogenation reaction according to an exemplary embodiment of the present application can obtain long-term stability and a high 1,3-butadiene yield, compared to a zinc ferrite-based catalyst in the related art, which is used for oxidative dehydrogenation of butene.

[Brief Description of Drawings]

FIG. 1 is a view schematically illustrating a catalyst for an oxidative dehydrogenation reaction according to an exemplary embodiment of the present application.
FIG. 2 is a view schematically illustrating a catalyst for an oxidative dehydrogenation reaction in the related art.
FIG. 3 is a view schematically illustrating a process by which coke is produced on the surface of a catalyst for an oxidative dehydrogenation reaction.

[Explanation of Reference Numerals and Symbols]

10: Carrier
20: First coating layer
30: Second coating layer
40: Catalyst coating layer

[Best Mode]

Hereinafter, the present application will be described in more detail.
In the specification, the 'butadiene' means 1,3-butadiene.
As described above, it is known that the activity of a ferrite-based catalyst having a spinel structure ($AFe_2O_4$) is good as a catalyst for a process of producing 1,3-butadiene through the oxidative dehydrogenation reaction of butene.
Meanwhile, it is known that a ferrite-based catalyst exhibits a result that is better than that of a bismuth-molybdenum catalyst (Mo-Bi catalyst) in terms of reactivity with 2-butene, particularly, trans-2-butene. Accordingly, even though the Mo-Bi catalyst is applied to the oxidative dehydrogenation reaction of 2-butene, an effect which is the same as that in the present application, that is, a result such as the conversion rate of butene or selectivity of butadiene is not obtained.
In this case, a $ZnFe_2O_4$ catalyst used in the oxidative dehydrogenation reaction of butene is generally produced by a co-precipitation method. In the co-precipitation, the $ZnFe_2O_4$ catalyst is prepared by subjecting the raw materials to precipitation, stirring, aging, washing, drying, and firing processes, and a step in which zinc (Zn) and ferrite (Fe) are homogeneously precipitated is very important.
Further, the zinc ferrite-based catalyst forms a spinel structure theoretically having a chemical formula of $ZnFe_2O_4$, and it is possible to add an additional function to the catalyst using a method for adding a metal other than Zn and Fe.
In addition, in the oxidative dehydrogenation reaction of butene for producing 1,3-butadiene, there is a problem in that the longer the catalytic reaction continues, the more the catalyst is deactivated due to the production of coke. In order to prevent the deactivation of such a catalyst and increase long-term stability, the present inventors have studied a method of adding a metal capable of helping with oxygen storage capacity, thermal stability, coke reduction, and the like to the catalyst, in consideration of oxygen diffusion, which is a major factor in oxidative dehydrogenation reactions.

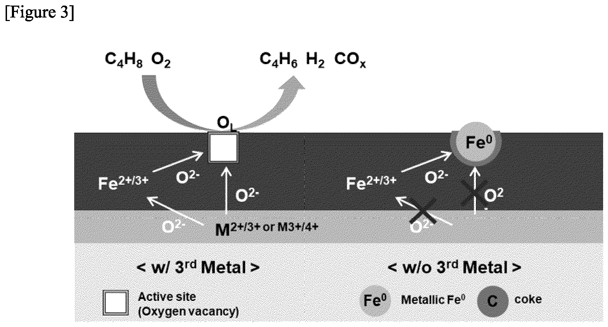

A process by which coke is produced on the surface of the catalyst for an oxidative dehydrogenation reaction is schematically illustrated in the following FIG. 3. As illustrated in the following FIG. 3, the Zn-Fe catalyst is reduced during the reaction to generate an oxygen vacancy and produce coke at the corresponding location, and the produced coke moves to become located at the support-catalyst interface. Thus, in an exemplary embodiment of the present application, a phenomenon in which coke is deposited on the catalyst may be suppressed, thereby providing a catalyst for an oxidative dehydrogenation reaction with excellent catalytic activity.

A catalyst for an oxidative dehydrogenation reaction according to an exemplary embodiment of the present application comprises: a carrier; a first coating layer provided on the carrier and comprising a metal oxide; and a second coating layer provided on the first coating layer and comprising a zinc ferrite-based catalyst, in which the metal oxide comprises one or more metals selected from among Ce, K, Mg, La and Y.

In an exemplary embodiment of the present application, the size and shape of the carrier may vary depending on the size of a reactor to be used for the reaction, and are not particularly limited, but the carrier may be spherical or cylindrical with a diameter of 2 mm to 20 mm.

In an exemplary embodiment of the present application, the carrier may have a porous structure. The carrier is not particularly limited as long as it has a porous structure, but may be a carrier having pores with a size enough to be provided with a first coating layer comprising the metal oxide and a second coating layer comprising the zinc ferrite-based catalyst.

Furthermore, according to an exemplary embodiment of the present application, the pore structure of the carrier may have a pore size of 10 $\mu$m to 300 $\mu$m, and a porosity of 10% to 50% relative to the total volume of the carrier.

As for the method of measuring the size of the pores on the surface of the carrier, a technique commonly used may be used, and even though it is not particularly limited, a mercury porosimeter may be used.

Further, according to an exemplary embodiment of the present application, the porous structure of the carrier may allow the carrier to have a specific surface area of 0.005 $m^2$/g to 0.5 $m^2$/g. The surface area of the carrier may typically be a BET surface area due to $N_2$ adsorption.

In an exemplary embodiment of the present application, the carrier may comprise one or more of alumina, silica, alumina/silica, silicon carbide, titania and zirconia. More specifically, the carrier may comprise alumina or alumina/silica, and has a porous structure in which the first coating layer and the second coating layer can be stably formed on the carrier, and a small number of regions which cause side reactions, thereby exhibiting chemical stability because the alumina or alumina/silica does not react with a zinc ferrite-based catalyst.

The catalyst for an oxidative dehydrogenation reaction according to an exemplary embodiment of the present application comprises a first coating layer provided on the carrier and comprising a metal oxide, in which the metal oxide comprises one or more metals selected from among Ce, K, Mg, La and Y.

When the metal oxide comprises K or Mg, coke which may be deposited on the catalyst may be effectively removed. In addition, when the metal oxide comprises Ce, La, or Y, the catalyst has a large oxygen storage capacity, so oxygen diffusion may be enhanced, and accordingly, the phenomenon of coke depositing on the catalyst may be suppressed.

The first coating layer may be provided on a portion of the surface of the carrier, and may be provided on the entire surface of the carrier. In order to more effectively suppress the phenomenon in which coke is deposited on the catalyst for an oxidative dehydrogenation reaction and improve the activity of the catalyst, the first coating layer is preferably provided on the entire surface of the carrier.

When the first coating layer comprises alumina ($Al_2O_3$) such as aluminum oxide, it is not possible to obtain the effect of suppressing the phenomenon in which coke is deposited on a catalyst as in the present invention because coke may be deposited on the alumina by the acid property of the alumina having acid sites. Therefore, according to an exemplary embodiment of the present application, the first coating layer does not comprise the alumina.

The catalyst for an oxidative dehydrogenation reaction according to an exemplary embodiment of the present application comprises a second coating layer provided on the first coating layer and comprising a zinc ferrite-based catalyst.

The second coating layer may be provided on a portion of the surface of the first coating layer, and may be provided on the entire surface of the first coating layer. In order to more effectively suppress the phenomenon in which coke is deposited on the catalyst for an oxidative dehydrogenation reaction and improve the activity of the catalyst, the second coating layer is preferably provided on the entire surface of the first coating layer.

In an exemplary embodiment of the present application, the zinc ferrite-based catalyst may be represented by the following Chemical Formula 1.

[Chemical Formula 1]        $ZnFe_xO_y$

In Chemical Formula 1,

x is 1 to 2.8, and
y is 1 to 6.

In Chemical Formula 1, x may be 1 to 2.8, 1.6 to 2.5, and preferably 1.8 to 2.2.

In an exemplary embodiment of the present application, a content of one or more metals selected from among Ce, K, Mg, La and Y may be 0.05 wt% to 5 wt%, 0.2 wt% to 0.8 wt%, and 0.3 wt% to 0.7 wt%, based on a total weight of the zinc ferrite-based catalyst. As described above, when the content of one or more metals selected from among Ce, K, Mg, La and Y satisfies the above-described content range, there is an effect of improving the butene conversion rate and the butadiene yield compared to a zinc ferrite-based catalyst in the related art. Furthermore, when the content of one or more metals selected from among Ce, K, Mg, La and Y is less than 0.05 wt%, it is difficult to expect that the performance of the catalyst is improved, and when the content exceeds 5 wt%, the reaction initiation temperature may be increased.

The catalyst for an oxidative dehydrogenation reaction according to an exemplary embodiment of the present application may be provided with a first coating layer comprising a metal oxide and a second coating layer comprising a zinc ferrite-based catalyst on a carrier in this order to suppress a phenomenon in which coke is deposited on the catalyst.

As described above, the Zn-Fe catalyst is reduced during the reaction to generate an oxygen vacancy and produce coke at the corresponding location, and the produced coke is grown and/or moves to become located at the support-catalyst interface. In particular, coke precursors that are weakly adsorbed on metals may move from the metal to the carrier, but in this case, the coke precursor may move and be located on the carrier with acidic properties such as alumina, and such coke precursors may degrade the activity and long-term stability of the catalyst. In contrast, the catalyst for an oxidative dehydrogenation reaction according to an exemplary embodiment of the present application may be provided with a first coating layer comprising a metal oxide and a second coating layer comprising a zinc ferrite-based catalyst on a carrier in this order to suppress a phenomenon in which coke is deposited on the catalyst and increase the activity of the catalyst.

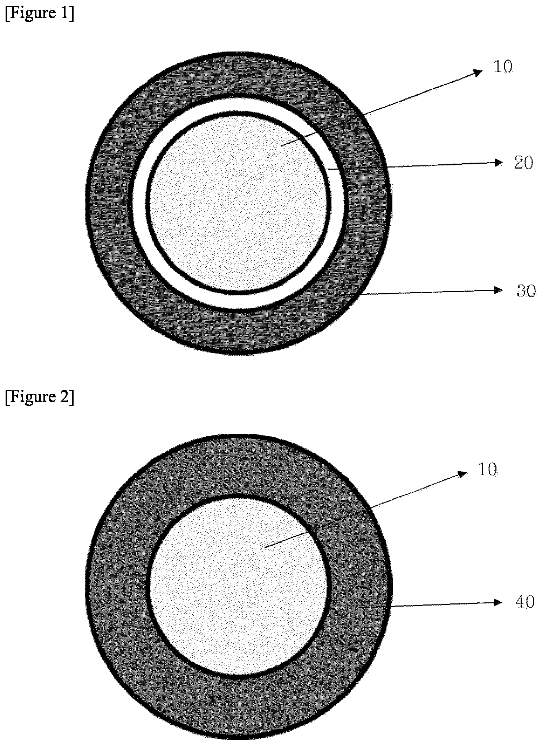

The catalyst for an oxidative dehydrogenation reaction according to an exemplary embodiment of the present application is schematically illustrated in the following FIG. 1, and a catalyst for an oxidative dehydrogenation reaction in the related art is schematically illustrated in FIG. 2.

As illustrated in the following FIG. 1, the catalyst for an oxidative dehydrogenation reaction according to an exemplary embodiment of the present application comprises: a carrier 10; a first coating layer 20 provided on the carrier 10 and comprising a metal oxide; and a second coating layer 30 provided on the first coating layer 20 and comprising a zinc ferrite-based catalyst, in which the metal oxide comprises one or more metals selected from among Ce, K, Mg, La and Y. Furthermore, as illustrated in the following FIG. 2, the catalyst for an oxidative dehydrogenation reaction in the related art comprises a carrier 10; and a catalyst coating layer 40.

Further, a method for producing a catalyst for an oxidative dehydrogenation reaction according to an exemplary embodiment of the present application comprises: forming a first coating layer by coating a carrier with a first solution comprising a metal oxide precursor, and then performing a first heat treatment process; and forming a second coating layer by coating the first coating layer with a second solution comprising a zinc ferrite-based catalyst, and then performing a second heat treatment process, in which the metal oxide precursor comprises one or more metals selected from among Ce, K, Mg, La and Y.

In the method for producing a catalyst for an oxidative dehydrogenation reaction according to an exemplary embodiment of the present application, the contents on the carrier, the first coating layer, the second coating layer, the zinc ferrite-based catalyst, and the like are the same as those described above.

The metal oxide precursor is a precursor of the metal oxide constituting the first coating layer, and there is no particular limitation in the metal oxide precursor, and ammonium salts, nitrates, carbonates, chlorides, and hydroxides, which comprise the one or more metals selected from among Ce, K, Mg, La and Y, or mixtures thereof may be applied in combination.

The first solution and the second solution may each independently further comprise a solvent, and as the solvent, a solvent known in the art may be applied. For example, the solvent may comprise water, but is not limited thereto.

As the coating method of the first solution and the second solution, methods known in the art may be used, and dip-coating, wash-coating and the like may be used, but the method is not limited thereto.

The first heat treatment process and the second heat treatment process may each independently comprise performing drying and firing. The drying may be performed at a temperature of 50°C to 150°C for 1 hour to 48 hours, and may be performed at a temperature of 60°C to 100°C for 5 hours to 36 hours, but is not limited thereto. Further, the firing may be performed at a temperature of 350°C to 1100°C for 1 hour to 10 hours in the air atmosphere, and may be performed at a temperature of 500°C to 1,000°C for 1.5 hours to 8 hours in the air atmosphere, but is not limited thereto.

The method for producing a catalyst for an oxidative dehydrogenation reaction according to an exemplary embodiment of the present application may additionally comprise preparing the zinc ferrite-based catalyst. The preparing of the zinc ferrite-based catalyst may comprise: obtaining a precipitate by bringing a metal precursor solution comprising a zinc precursor, a ferrite precursor, and water into contact with an aqueous basic solution; and filtering the precipitate, and then drying the filtered precipitate, and firing the dried precipitate.

A content of the zinc precursor may be 0.1 wt% to 5 wt% and 0.1 wt% to 3 wt%, based on a total weight of the metal precursor solution. In addition, a content of the ferrite precursor may be 1 wt% to 10 wt% and 1 wt% to 7 wt%, based on a total weight of the metal precursor solution. When the contents of the zinc precursor and the ferrite precursor satisfy the above-described ranges, a zinc ferrite-based catalyst is easily synthesized during the formation of a precipitate by the co-precipitation method.

The zinc precursor and the ferrite precursor may be each independently one or more selected from the group consisting of nitrate, ammonium salt, sulfate, and chloride, or a hydrate thereof. Specifically, it is preferred that the zinc precursor and the ferrite precursor are nitrate or chloride, or a hydrate thereof.

The zinc precursor may be zinc chloride ($ZnCl_2$). In this case, the formation of the zinc ferrite-based catalyst is excellent. The ferrite precursor may be ferric chloride hydrate ($FeCl_3 \cdot 6H_2O$). In this case, the formation of the zinc ferrite-based catalyst is excellent.

As the water, pure water (DI water) may be used. The temperature of the pure water (DI water) may be more than 0°C and 40°C or less. Preferably, the temperature of the pure water may be more than 0°C and 30°C or less. The temperature of the pure water may be more preferably 5°C to 25°C. When the temperature of the pure water satisfies the above range, the selectivity and yield of butadiene according to the oxidative dehydrogenation reaction may be ultimately improved by increasing the amount of catalyst produced by the precipitation and adjusting the content of the active catalyst.

A pH of the aqueous basic solution may be 7 to 10. Preferably, the pH may be 7.5 to 9. When the pH satisfies the above range, there is an effect of stably producing the zinc ferrite catalyst.

The aqueous basic solution may be one or more selected from the group consisting of potassium hydroxide, ammonium carbonate, ammonium hydrogen carbonate, a sodium hydroxide aqueous solution, a sodium carbonate aqueous solution, and ammonia water. Preferably, the aqueous basic solution may be ammonia water. In this case, in the process of producing a zinc ferrite-based catalyst, there is an effect that facilitates precipitation, and thus makes the formation of catalyst particles excellent.

A concentration of the aqueous basic solution may be 20 wt% to 40 wt%, and 25 wt% to 30 wt%.

The obtaining of the precipitate may further comprise bringing the metal precursor solution into contact with the aqueous basic solution, and then stirring the resulting solution. The formation of precipitations of metal precursors is facilitated by further comprising the stirring of the resulting solution, so that catalyst particles are advantageously formed. The stirring of the resulting solution may be performed at room temperature, and the method for stirring the resulting solution may be used without limitation as long as the method mixes a liquid with a liquid. Further, the stirring time of the stirring of the resulting solution may be 30 minutes to 3 hours, and may be 1 hour to 2 hours.

The filtering of the precipitate is not particularly limited as long as the filtration method is typically used in the art. For example, the method may be vacuum filtration. Specifically, the method may be a method of filtering the precipitate by using a vacuum pump to reduce pressure, and in this case, there are effects of washing and separating the catalyst from moisture.

The method may further comprise washing the precipitate before the precipitate is filtered and then fired. Unnecessary ions remaining in the precipitate may be removed by further comprising the washing of the precipitate.

The drying of the precipitate may be performed before the precipitate is fired after the precipitate is filtered and then washed. The drying of the precipitate is not particularly limited as long as the drying method is typically used in the art. For example, a dryer may be used, and an oven may be used. The drying of the precipitate may be performed at 80°C to 150°C.

The firing of the precipitate may maintain 600°C to 800°C for 5 hours to 10 hours by increasing the temperature at a rate of 1°C/min at 80°C. In the firing of the precipitate, the precipitate may be fired specifically at 600°C to 700°c, and more specifically at 600°C to 650°C. In the firing of the precipitate, the precipitate may be fired specifically for 5 hours to 8 hours, and more specifically for 5 hours to 6 hours.

The method for firing the precipitate may be a heat treatment method typically used in the art.

Further, an exemplary embodiment of the present application provides a method for producing butadiene, the method comprising: preparing the catalyst for an oxidative dehydrogenation reaction; and producing butadiene by using the catalyst for an oxidative dehydrogenation reaction in an oxidative dehydrogenation reaction of butene.

In an exemplary embodiment of the present application, the producing of the butadiene may allow a raw material comprising C4 fractions, steam, oxygen ($O_2$), and nitrogen ($N_2$) to react at a gas hourly space velocity (GHSV) of 200 $h^{-1}$ to 400 $h^{-1}$ under the conditions of a reaction temperature of 400°C to 600°C and a pressure of 0.1 bar to 10 bar.

The C4 fractions may mean C4 raffinate-1,2,3 remaining by separating useful compounds from a C4 mixture produced by naphtha cracking, and may mean C4 classes which may be obtained through ethylene dimerization.

In an exemplary embodiment of the present application, the C4 fractions may be one or a mixture of two or more selected from the group consisting of n-butane, trans-2-butene, cis-2-butene, and 1-butene.

In an exemplary embodiment of the present application, the steam or nitrogen ($N_2$) is a diluted gas introduced for the purpose of reducing the explosion danger of the reactant, preventing coking of the catalyst, removing the reaction heat, and the like, in the oxidative dehydrogenation reaction.

In an exemplary embodiment of the present application, the oxygen ($O_2$) is an oxidant and reacts with C4 fractions to cause a dehydrogenation reaction.

In an exemplary embodiment of the present application, the oxidative dehydrogenation reaction may be performed according to the following Reaction Formula 1 or Reaction Formula 2.

$$[\text{Reaction Scheme 1}] \qquad C_4H_8 + 1/2O_2 \rightarrow C_4H_6 + H_2O$$

$$[\text{Reaction Scheme 2}] \qquad C_4H_{10} + O_2 \rightarrow C_4H_6 + 2H_2O$$

Hydrogen of butane or butene is removed by the oxidative dehydrogenation reaction, and as a result, butadiene is produced. Meanwhile, the oxidative dehydrogenation reaction may produce a side reaction product comprising carbon monoxide (CO), carbon dioxide ($CO_2$), or the like, in addition to the main reaction such as Reaction Formula 1 or 2. The oxidative dehydrogenation reaction may comprise a process in which the side reaction product is separated so as not to be continuously accumulated in the process, and is released out of the system.

[Mode for Invention]

Hereinafter, the present application will be described in detail with reference to Examples for specifically describing the present application. However, the Examples according to the present application may be modified in various forms, and it is not interpreted that the scope of the present application is limited to the Examples described in detail below. The Examples of the present application are provided for more completely explaining the present application to the person with ordinary skill in the art.

**<Examples>**

**<Example 1> Supporting 0.5 wt% of Ce**

**1) Production of zinc ferrite-based catalyst**

A metal precursor solution was prepared by dissolving 12.019 g of zinc chloride ($ZnCl_2$) and 47.662 g of ferric chloride ($FeCl_3$) in 155.59 g of distilled water. In this case, for a molar ratio of the metal components comprised in the metal precursor solution, Zn : Fe was 1:2. An aqueous ammonia solution was added dropwise to the prepared aqueous metal precursor solution such that the pH was 9, and the resulting solution was stirred for 1 hour and co-precipitated. Thereafter, a co-precipitate was obtained by filtering the co-precipitation solution under reduced pressure, and after the co-precipitate was dried at 90°C for 16 hours, and then the temperature was increased up to 650°C at a heating rate of 1°C/min at 80°C in the air atmosphere, a zinc-iron oxide ($ZnFe_2O_4$) powder having a spinel structure was produced by maintaining the temperature for 6 hours.

**2) Preparation of Ce precursor solution**

A Ce precursor solution was prepared by diluting $Ce(NO_3)_3 \cdot 6H_2O$ with water (200 mL), such that the weight of Ce became 0.5 wt% based on the weight of the zinc-iron oxide ($ZnFe_2O_4$) powder.

**3) Production of first coating layer comprising metal oxide**

After a porous aluminum silicate carrier was immersed in the Ce precursor solution prepared above, water was evaporated under reduced pressure conditions at 90°C. The carrier on which the Ce precursor was supported was dried at 120°C for 16 hours. Thereafter, the temperature was increased from 80°C to 650°C at a heating rate of 1°C/min in the air atmosphere, and maintained for 6 hours to produce a carrier on which the first coating layer comprising Ce oxide was formed.

**4) Production of catalyst for oxidative dehydrogenation reaction**

The zinc-iron oxide powder prepared above was ground to a size of 180 $\mu$m or less and diluted with water to prepare a catalyst slurry. The carrier on which the first coating layer comprising Ce oxide was formed was immersed in the catalyst slurry, and the resulting mixture was aerated, and then dried at 120°C for 1 hour. Thereafter, the process of immersing the dried porous aluminum silicate carrier again in the catalyst slurry, and aerating and then drying the resulting mixture was repeated three times. A catalyst for an oxidative dehydrogenation reaction having a porous structure was produced by drying the thus obtained catalyst at 120°C for 16 hours, increasing the temperature up to 650°C at a heating rate of 1°C/min at 80°C in the air atmosphere, and then maintaining the temperature for 6 hours.

**<Example 2> Supporting 0.5 wt% of La**

A process was performed in the same manner as in Example 1, except that a La precursor solution was applied instead of the Ce precursor solution. In this case, as the La precursor solution, a La precursor solution was prepared by diluting $La(NO_3)_3$ with water such that the weight of La became 0.5 wt% based on the weight of the zinc-iron oxide ($ZnFe_2O_4$) powder.

**<Example 3> Supporting 0.5 wt% of K**

A process was performed in the same manner as in Example 1, except that a K precursor solution was applied instead of the Ce precursor solution. In this case, as the K precursor solution, a K precursor solution was prepared by diluting $KNO_3$ with water such that the weight of K became 0.5 wt% based on the weight of the zinc-iron oxide ($ZnFe_2O_4$) powder.

**<Example 4> Supporting 0.5 wt% of Mg**

A process was performed in the same manner as in Example 1, except that a Mg precursor solution was applied instead of the Ce precursor solution. In this case, as the Mg precursor solution, a Mg precursor solution was prepared by diluting $Mg(NO_3)_2$ with water such that the weight of Mg became 0.5 wt% based on the weight of the zinc-iron oxide ($ZnFe_2O_4$) powder.

**<Example 5> Supporting 0.5 wt% of Y**

A process was performed in the same manner as in Example 1, except that a Y precursor solution was applied instead of the Ce precursor solution. In this case, as the Y precursor solution, a Y precursor solution was prepared by diluting $Y(NO_3)_3$ with water such that the weight of Y became 0.5 wt% based on the weight of the zinc-iron oxide ($ZnFe_2O_4$) powder.

**<Comparative Example 1>**

**1) Production of zinc ferrite-based catalyst**

Zinc-iron oxide ($ZnFe_2O_4$) powder was prepared in the same manner as in the production of the zinc ferrite-based catalyst in Example 1.

**2) Production of catalyst for oxidative dehydrogenation reaction**

The zinc-iron oxide powder prepared above was ground to a size of 180 $\mu$m or less and diluted with water at a weight ratio of 1 : 1 to prepare a catalyst slurry. A porous aluminum silicate carrier was immersed in the produced catalyst slurry, and the resulting mixture was aerated, and then dried at 120°C for 1 hour. Thereafter, the process of immersing the dried porous aluminum silicate carrier again in the catalyst slurry, and aerating and then drying the resulting mixture was repeated three times. A catalyst for an oxidative dehydrogenation reaction having a porous structure was produced by drying the thus obtained catalyst at 120°C for 16 hours, increasing the temperature up to 650°C at a heating rate of 1°C/min at 80°C in the air atmosphere, and then maintaining the temperature for 6 hours.

<Comparative Example 2> Adding 0.5 wt% of Ce

1) Production of zinc ferrite-based catalyst

Zinc-iron oxide ($ZnFe_2O_4$) powder was prepared in the same manner as in the production of the zinc ferrite-based catalyst in Example 1.

2) Production of catalyst for oxidative dehydrogenation reaction

A catalyst for an oxidative dehydrogenation reaction was produced in the same manner as in Comparative Example 1, except that $Ce(NO_3)_3 \cdot 6H_2O$ was additionally added such that during the preparation of the catalyst slurry, the weight of Ce became 0.5 wt% based on the weight of the zinc-iron oxide ($ZnFe_2O_4$) powder.

**<Comparative Example 3> Addition of 0.5 wt% of La**

**1) Production of zinc ferrite-based catalyst**

Zinc-iron oxide ($ZnFe_2O_4$) powder was prepared in the same manner as in the production of the zinc ferrite-based catalyst in Example 1.

**2) Production of catalyst for oxidative dehydrogenation reaction**

A catalyst for an oxidative dehydrogenation reaction was produced in the same manner as in Comparative Example 1, except that $La(NO_3)_3$ was additionally added such that during the preparation of the catalyst slurry, the weight of La became 0.5 wt% based on the weight of the zinc-iron oxide ($ZnFe_2O_4$) powder.

**<Experimental Example 1> Analysis of catalyst for oxidative dehydrogenation reaction**

The analysis of contents of the metal, and the like in the catalysts for an oxidative dehydrogenation reaction produced in the Examples and the Comparative Examples may be performed using an inductively coupled plasma (ICP) analysis. The ICP analysis may be performed using an inductively coupled plasma-optical emission (ICP-OES) apparatus. More specifically, an ICP-OES (Optima 7300DV) device may be used, and the procedure is as follows.

<Experimental Method 1, Analysis of main components (Fe, Zn)>

1) About 0.01 g of the sample is aliquoted into a platinum crucible and the weight is accurately measured.
2) 3 mL of concentrated hydrochloric acid is added thereto and the resulting mixture is well shaken and mixed.
3) The sample is heated and decomposed (130°C, 3 hr).
4) When the sample is dissolved, the sample is cooled to room temperature, and then 500 µL of a Sc internal standard solution is added thereto, and the resulting mixture is diluted with ultrapure water such that a total volume becomes 50 mL.
5) The sample is measured by ICP-OES. When the weight exceeds the calibration range, the sample is additionally diluted and measured.

<Experimental Method 2, trace analysis>

1) About 0.1 g of the sample is aliquoted into a platinum crucible and the weight is accurately measured.
2) 3 mL of concentrated hydrochloric acid is added thereto and the resulting mixture is well shaken and mixed.
3) The sample is heated and decomposed (130°C, 3 hr).
4) When the sample is dissolved, the sample is cooled to room temperature, and then 100 µL of a Sc internal standard solution is added thereto, and the resulting mixture is diluted with ultrapure water such that a total volume becomes 10 mL.
5) The sample is measured by ICP-OES. When the weight exceeds the calibration range, the product is additionally diluted and measured.

**<Experimental Example 2> Production of butadiene**

Under the conditions of 300°C to 600°C, GHSV = 50 h$^{-1}$ to 200 h$^{-1}$, OBR = 0.5 to 1.5, SBR = 6 to 10, and NBR = 1, 1,3-butadiene was produced from the oxidative dehydrogenation reaction by using the zinc ferrite-based catalysts prepared in the Examples and the Comparative Examples.

$$OBR = Oxygen/total\ 2\text{-}butene\ ratio$$

$$SBR = Steam/total\ 2\text{-}butene\ ratio$$

$$NBR = Nitrogen/total\ 2\text{-}butene\ ratio$$

In addition, in the oxidative dehydrogenation reaction of butene, butene conversion rate, butadiene selectivity, butadiene yield, and the like were measured, and are shown in the following Table 1.
In the present specification, the 'yield (%)' is defined as a value obtained by dividing the number of moles of 1,3-butadiene, which is a product of an oxidative dehydrogenation reaction, by the number of moles of butene which is a raw material. For example, the yield may be represented by the following equation.

Yield (%) = [(the number of moles of 1,3-butadiene produced)/(the number of moles of butene supplied)} x 100

In the present specification, the 'conversion rate (%)' refers to a rate at which a reactant is converted into a product, and for example, the conversion rate of butene may be defined by the following equation.

Conservion rate (%) = [(the number of moles of butene reacted)/(the number of moles of butene supplied)] x 100

In the present specification, the 'selectivity (%)' is defined as a value obtained by dividing the change amount of butadiene (BD) by the change amount of butene (BE). For example, the selectivity may be represented by the following equation.

Selectivity (%) = [(the number of moles of 1,3-butadiene or COx produced)/(the number of moles of butene reacted)] x 100

**<Experimental Example 3> Analysis of amount of coke produced**

In order to measure the amount of coke produced from the catalysts of the Examples and the Comparative Examples recovered after the reaction, thermogravimetric analysis was performed using TGA 550 manufactured by Waters. The analysis conditions were a heating rate of 10°C/min from 50°C to 650°C, a flow rate of air 60 cm$^3$/min, and a balance of N$_2$ 40 cm$^3$/min in the air atmosphere. The amount of coke produced and the coke reduction rate were measured, and are shown in the following Table 1.
In the present specification, the amount (wt%) of coke produced is defined as a value indicating the amount of weight change according to the TGA analysis results. For example, the amount of coke produced may be represented by the following equation.

Amount (wt%) of coke produced = [(weight of catalyst before TGA analysis - weight of catalyst after TGA analysis)/(weight of catalyst before TGA analysis)] $\times$ 100

In this specification, the coke reduction rate (%) is defined as a value indicating the amount of change in the amount of coke produced. For example, the coke reduction rate may be represented by the following equation.

Coke reduction rate (%) = [(amount of coke produced in Comparative Example 1 - amount of coke produced in Example or Comparative Example)/amount of coke produced in Comparatvie Example 1)] $\times$ 100

[Table 1]

| | Butene conversion rate (%) | Butadiene selectivity (%) | Butadiene yield (%) | COx selectivity (%) | Amount of coke produced (wt%) | Coke reduction rate (%) |
|---|---|---|---|---|---|---|
| Example 1 | 86.4 | 86.6 | 74.8 | 12.5 | 0.0050 | 59 |
| Example 2 | 83.5 | 85.8 | 71.9 | 12.1 | 0.0063 | 48 |
| Example 3 | 94.5 | 88.4 | 83.5 | 8.0 | 0.0040 | 67 |
| Example 4 | 79.1 | 86.2 | 68.2 | 13.2 | 0.0052 | 57 |
| Example 5 | 72.5 | 80.4 | 58.3 | 16.3 | 0.0101 | 17 |
| Comparative Example 1 | 78.8 | 86.2 | 67.9 | 12.5 | 0.0122 | - |
| Comparative Example 2 | 80.7 | 84.8 | 68.5 | 14.0 | 0.0130 | -7 |
| Comparative Example 3 | 72.9 | 84.1 | 61.3 | 15.6 | 0.0137 | -12 |

As shown in the above results, it can be confirmed that the catalysts for an oxidative dehydrogenation reaction of Examples 1 to 5 of the present application produced less coke and had the better coke reduction rate than the catalysts for an oxidative dehydrogenation reaction of Comparative Examples 1 to 3. In particular, when Example 1 is compared with Comparative Example 2, it can be confirmed that forming an additional metal oxide in the first coating layer on the catalyst for an oxidative dehydrogenation reaction may not only suppress a phenomenon in which coke is deposited on the catalyst, but also increase the activity of the catalyst, as in the following FIG. 1.

A catalyst for an oxidative dehydrogenation reaction according to an exemplary embodiment of the present application can be provided with a first coating layer comprising a metal oxide and a second coating layer comprising a zinc ferrite-based catalyst on a carrier in this order to suppress a phenomenon in which coke is deposited on the catalyst and increase the activity of the catalyst.

Furthermore, the catalyst for an oxidative dehydrogenation reaction according to an exemplary embodiment of the present application can obtain long-term stability and a high 1,3-butadiene yield, compared to a zinc ferrite-based catalyst in the related art, which is used for oxidative dehydrogenation of butene.

**Claims**

1. A catalyst for an oxidative dehydrogenation reaction, comprising:

    a carrier;
    a first coating layer provided on the carrier and comprising a metal oxide; and
    a second coating layer provided on the first coating layer and comprising a zinc ferrite-based catalyst,
    wherein the metal oxide comprises one or more metals selected from among Ce, K, Mg, La and Y.

2. The catalyst of claim 1, wherein the first coating layer is provided on the entire surface of the carrier.

3. The catalyst of claim 1, wherein the second coating layer is provided on the entire surface of the first coating layer.

4. The catalyst of claim 1, wherein the zinc ferrite-based catalyst is represented by the following Chemical Formula 1:

    [Chemical Formula 1]          $ZnFe_xO_y$

    in Chemical Formula 1,

    x is 1 to 2.8, and
    y is 1 to 6.

5. The catalyst of claim 1, wherein a content of the one or more metals selected from among Ce, K, Mg, La and Y is 0.05 wt% to 5 wt% based on a total weight of the zinc ferrite-based catalyst.

6. The catalyst of claim 1, wherein the carrier comprises one or more of alumina, silica, alumina/silica, silicon carbide, titania, and zirconia.

7. A method for producing a catalyst for an oxidative dehydrogenation reaction, the method comprising:

forming a first coating layer by coating a carrier with a first solution comprising a metal oxide precursor, and then performing a first heat treatment process; and
forming a second coating layer by coating the first coating layer with a second solution comprising a zinc ferrite-based catalyst, and then performing a second heat treatment process,
wherein the metal oxide precursor comprises one or more metals selected from among Ce, K, Mg, La and Y.

8. The method of claim 7, wherein the zinc ferrite-based catalyst is represented by the following Chemical Formula 1:

$$[\text{Chemical Formula 1}] \qquad ZnFe_xO_y$$

in Chemical Formula 1,

x is 1 to 2.8, and
y is 1 to 6.

9. A method for producing butadiene, the method comprising:

preparing the catalyst for an oxidative dehydrogenation reaction of any one of claims 1 to 6; and
producing butadiene using the catalyst for an oxidative dehydrogenation reaction in an oxidative dehydrogenation reaction of butene.

[Figure 1]

[Figure 2]

[Figure 3]

**INTERNATIONAL SEARCH REPORT**

| International application No. |
|---|
| **PCT/KR2023/013694** |

### A. CLASSIFICATION OF SUBJECT MATTER

**B01J 35/00**(2006.01)i; **B01J 23/10**(2006.01)i; **B01J 23/80**(2006.01)i; **B01J 37/02**(2006.01)i; **B01J 37/03**(2006.01)i; **B01J 21/04**(2006.01)i; **B01J 21/08**(2006.01)i; **B01J 21/06**(2006.01)i; **B01J 37/08**(2006.01)i

According to International Patent Classification (IPC) or to both national classification and IPC

### B. FIELDS SEARCHED

Minimum documentation searched (classification system followed by classification symbols)

B01J 35/00(2006.01); B01J 21/04(2006.01); B01J 23/56(2006.01); B01J 23/80(2006.01); B01J 23/887(2006.01); B01J 35/02(2006.01); B01J 37/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 1,3-부타디엔(1,3-butadiene), 촉매(catalyst), 마그네슘(magnesium), 담체(carrier), 페라이트(ferrite)

### C. DOCUMENTS CONSIDERED TO BE RELEVANT

| Category* | Citation of document, with indication, where appropriate, of the relevant passages | Relevant to claim No. |
|---|---|---|
| Y | KR 10-2021-0059424 A (LG CHEM, LTD.) 25 May 2021 (2021-05-25)<br>See claims 1-11; and paragraphs [0077]-[0078]. | 1-9 |
| Y | KR 10-2020-0091014 A (HYOSUNG CHEMICAL CORPORATION) 30 July 2020 (2020-07-30)<br>See claim 1; and paragraphs [0025]-[0064]. | 1-9 |
| A | KR 10-0387433 B1 (HYOSUNG CORPORATION) 18 June 2003 (2003-06-18)<br>See entire document. | 1-9 |
| A | JP 2016-500333 A (BASF SE) 12 January 2016 (2016-01-12)<br>See entire document. | 1-9 |
| A | KR 10-2021-0018566 A (HYOSUNG CHEMICAL CORPORATION) 18 February 2021 (2021-02-18)<br>See entire document. | 1-9 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **03 January 2024** | **03 January 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/013694**

| Patent document cited in search report | | | Publication date (day/month/year) | Patent family member(s) | | | Publication date (day/month/year) |
|---|---|---|---|---|---|---|---|
| KR | 10-2021-0059424 | A | 25 May 2021 | None | | | |
| KR | 10-2020-0091014 | A | 30 July 2020 | KR | 10-2162079 | B1 | 07 October 2020 |
| KR | 10-0387433 | B1 | 18 June 2003 | KR | 10-2002-0048142 | A | 22 June 2002 |
| JP | 2016-500333 | A | 12 January 2016 | CN | 104955569 | A | 30 September 2015 |
| | | | | EP | 2928603 | A1 | 14 October 2015 |
| | | | | KR | 10-2015-0091387 | A | 10 August 2015 |
| | | | | WO | 2014-086813 | A1 | 12 June 2014 |
| KR | 10-2021-0018566 | A | 18 February 2021 | KR | 10-2223637 | B1 | 09 March 2021 |

Form PCT/ISA/210 (patent family annex) (July 2022)

**EP 4 417 308 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220158634